(19) Europäisches Patentamt
European Patent Office
Office européen des brevets

(11) Publication number: 0 277 917 A2

(12) # EUROPEAN PATENT APPLICATION

(21) Application number: 88810050.0

(22) Date of filing: 29.01.88

(51) Int. Cl.$^4$: C 07 H 19/173
A 61 K 31/70

(30) Priority: 04.02.87 US 11169

(43) Date of publication of application:
10.08.88 Bulletin 88/32

(84) Designated Contracting States:
AT BE CH DE ES FR GB GR IT LI LU NL SE

(71) Applicant: CIBA-GEIGY AG
Klybeckstrasse 141
CH-4002 Basel (CH)

(72) Inventor: Hutchison, Alan J.
35 Lynwood Road
Verona New Jersey 07044 (US)

A request for correction of the description and claim 10 has been filed pursuant to Rule 88 EPC. A decision on the request will be taken during the proceedings before the Examining Division (Guidelines for Examination in the EPO, A-V, 2.2).

(54) **Certain adenosine 5'-carboxamide derivatives.**

(57) The compounds of the formula I

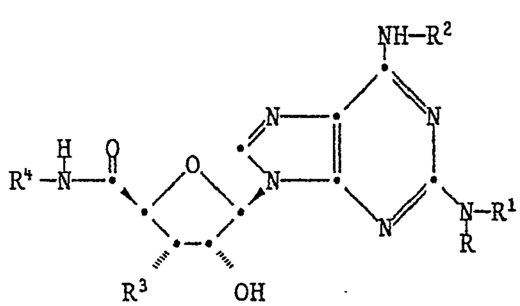

(I)

wherein R represents hydrogen or lower alkyl; $R^1$ represents cycloalkyl, cycloalkyl-lower alkyl, 2-norbornanyl, 2-norbornanyl-lower alkyl, aryl, aryl-lower alkyl, aryl-cycloalkyl, 9-fluorenyl, diaryl-lower alkyl or 9-fluorenyl-lower alkyl; or $R^1$ represents a bicyclic benzo-fused 5- or 6-membered saturated carbocyclic radical or a benzo-fused 5- or 6-membered saturated heterocyclic radical containing a heteroatom selected from oxygen and sulfur which is directly attached to the fused benzene ring, any said bicyclic radical being unsubstituted or substituted on the benzo portion by lower alkyl, lower alkoxy, hydroxy, halogen or trifluoromethyl, or by a substituent -W-Z in which W represents a direct bond, lower alkylene, lower alkenylene, thio-lower alkylene or oxy-lowe alkylene and Z represents cyano, carboxy or carboxy derivatized in the form of a pharmaceutically acceptable ester or amide; $R^2$ represents hydrogen, lower alkyl or aryl-lower alkyl; $R^3$ represents hydrogen or hydroxy; $R^4$ represents hydrogen, lower alkyl, aryl-lower alkyl, cycloalkyl or hydroxy-lower alkyl; pharmaceutically acceptable ester derivatives thereof in which free hydroxy groups are esterified in form of a pharmaceutically acceptable ester; pharmaceutically acceptable salts thereof; their preparation, and their use as adenosine-2 receptor agonists are disclosed.

EP 0 277 917 A2

**Description**

Certain adenosine 5′-carboxamide derivatives

The instant invention is directed to certain 2-substituted adenosine-5′-carboxamide derivatives as adenosine receptor ligands, to pharmaceutical compositions thereof, to methods for their preparation, and to their use in mammals as therapeutically effective adenosine receptor agonists.

The compounds of the invention are effective as adenosine, particularly adenosine-2 (A-2) receptor ligands which are useful in mammals as adenosine receptor agonists, particularly as adenosine-2 (A-2) receptor agonists.

Said advantageous properties render the compounds of the invention useful for the treatment of conditions in mammals responsive to selective adenosine receptor stimulation, particularly to adenosine-2 receptor stimulation, e.g. cardiovascular conditions such as hypertension, thrombosis and atherosclerosis, also central nervous system conditions comprising psychotic conditions such as schizophrenia, and convulsive disorders such as epilepsy.

More specifically, the instant invention is directed to the compounds of the formula I

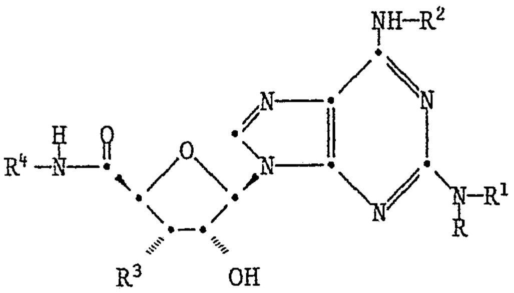

(I)

wherein R represents hydrogen or lower alkyl; $R^1$ represents cycloalkyl, cycloalkyl-lower alkyl, 2-norbornanyl, 2-norbornanyl-lower alkyl, aryl, aryl-lower alkyl, aryl-cycloalkyl, 9-fluorenyl, diaryl-lower alkyl or 9-fluorenyl-lower alkyl; or $R^1$ represents a bicyclic benzo-fused 5- or 6-membered saturated carbocyclic radical or a benzo-fused 5- or 6-membered saturated heterocyclic radical containing a heteroatom selected from oxygen and sulfur which is directly attached to the fused benzene ring, any said bicyclic radical being unsubstituted or substituted on the benzo portion by lower alkyl, lower alkoxy, hydroxy, halogen or trifluoromethyl, or by a substituent -W-Z in which W represents a direct bond, lower alkylene, lower alkenylene, thio-lower alkylene or oxy-lower alkylene and Z represents cyano, carboxy or carboxy derivatized in the form of a pharmaceutically acceptable ester or amide; $R^2$ represents hydrogen, lower alkyl or aryl-lower alkyl; $R^3$ represents hydrogen or hydroxy; $R^4$ represents hydrogen, lower alkyl, aryl-lower alkyl, cycloalkyl or hydroxy-lower alkyl; pharmaceutically acceptable ester derivatives thereof in which free hydroxy groups are esterified in form of a pharmaceutically acceptable ester; and pharmaceutically acceptable salts thereof.

Aryl hereinbefore and hereinafter represents an optionally substituted carbocyclic aromatic radical, being preferably 1- or 2-naphthyl, phenyl, or naphthyl or phenyl substituted by one to three of lower alkyl, lower alkoxy, hydroxy, halogen or trifluoromethyl, or naphthyl or phenyl substituted by a substituent -W-Z in which W represents a direct bond, lower alkylene, lower alkenylene, thio-lower alkylene or oxy-lower alkylene and Z represents cyano, carboxy or carboxy derivatized in the form of a pharmaceutically acceptable ester or amide; or aryl represents a heterocyclic aromatic radical, being preferably pyridyl or thienyl, each optionally substituted as described above for phenyl.

A subembodiment relates to compounds of formula I wherein all residues have meaning as defined hereinbefore except that R is different from lower alkyl, $R^1$ is different from cycloalkyl, W is different from lower alkenylene, and, within aryl, naphthyl, pyridyl and thienyl are exclusively unsubstituted.

Preferred are the compounds of formula I wherein R represents hydrogen or lower alkyl; $R^1$ represents cylcoalkyl-lower alkyl; or $R^1$ represents aryl-lower alkyl wherein aryl represents pyridyl, thienyl, naphthyl, phenyl, phenyl substituted by one or two substituents selected from halogen, trifluoromethyl, lower alkoxy, hydroxy and lower alkyl, or phenyl substituted by a substituent -W-Z in which W represents a direct bond, lower alkylene, lower alkenylene, thio-lower alkylene or oxy-lower alkylene, and Z represents cyano, carboxy, carboxy derivatized in the form of a pharmaceutically acceptable ester or carboxy derivatized in the form of a pharmaceutically acceptable amide; or $R^1$ represents a substituent of the formula B

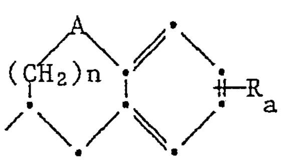

(B)

in which A represents methylene, oxygen or sulfur, n represents zero or one, and $R_a$ represents hydrogen, lower alkyl, lower alkozy, halogen or -W-Z as defined above; $R^2$ represents hydrogen or lower alkyl; $R^3$ represents hydrogen or hydroxy; $R^4$ represents hydrogen, lower alkyl, cycloalkyl, hydroxy-lower alkyl, or aryl-lower alkyl in which aryl represents pyridyl, thienyl or phenyl; pharmaceutically acceptable ester derivatives thereof in which one or more free hydroxy groups are esterified in form of a pharmaceutically acceptable ester; and pharmaceutically acceptable salts thereof.

A subembodiment relates to compounds of formula I being disclosed hereinbefore as being preferred wherein all residues have meaning as defined hereinbefore except that R is different from lower alkyl, $R^1$ is different from cycloalkyl-lower alkyl and naphthyl; and W is different from lower alkenylene.

Preferred are the compounds of formula (Ia)

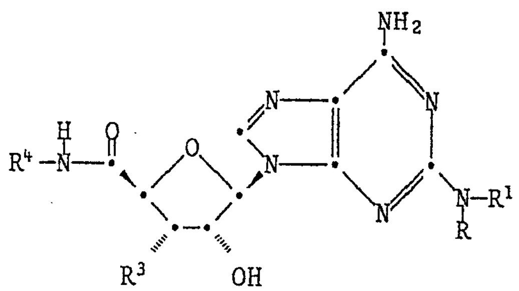

(Ia)

wherein R represents hydrogen or lower alkyl; $R^1$ represents cycloalkyl-lower alkyl; or $R^1$ represents aryl-lower aryl in which aryl represents thienyl, pyridyl, phenyl or phenyl monosubstituted by halogen, trifluoromethyl, lower alkoxy, hydroxy, lower alkyl, or by a substituent -W-Z in which W represents a direct bond, lower alkylene, thio-lower alkylene or oxy-lower alkylene, and Z represents cyano, carboxy, lower alkoxycarbonyl, carbamoyl, N-mono- or N,N-di-lower alkylcarbamoyl; or $R^1$ represents a substituent of the formula B′

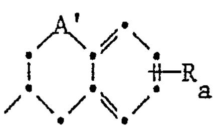

(B')

in which A′ represents a direct bond, methylene, oxygen or sulfur, and $R_a$ represents hydrogen, lower alkyl, lower alkoxy, hydroxy, halogen or -W-Z as defined above; $R^3$ represents hydrogen or hydroxy; and $R^4$ represents hydrogen, lower alkyl, cycloalkyl or hydroxy-lower alkyl; pharmaceutically acceptable prodrug ester derivatives thereof in which one or more free hydroxy groups are esterified in form of a pharmaceutically acceptable ester; and pharmaceutically acceptable salts thereof.

A subembodiment relates to compounds of formula Ia wherein all residues have meaning as defined hereinabove except that R is different from lower alkyl, and $R^1$ is different from cycloalkyl-lower alkyl and thienyl.

Preferred are the said compounds of formula I and Ia wherein $R^3$ represents hydroxy, and ester derivatives thereof.

Particularly preferred are the compounds of formula Ia wherein $R^3$ represents hydroxy; $R^4$ represents lower alkyl, cyclopropyl or hydroxy-lower alkyl; and R, $R^1$, A′ and $R_a$ having meaning as defined above; pharmaceutically acceptable prodrug ester derivatives thereof in which free hydroxy groups are esterified in form of a pharmaceutically acceptable ester; and pharmaceutically acceptable salts thereof.

Further preferred are the compounds of formula II

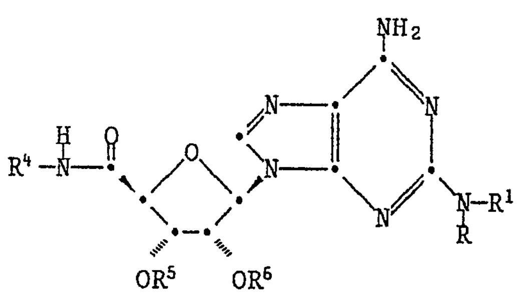

(II)

wherein R represents hydrogen or $C_1$-$C_4$-alkyl; $R^1$ represents ($C_5$- or $C_6$)-cycloalkyl-$C_1$-$C_4$-alkyl, or $R^1$ represents aryl-$C_1$-$C_4$-alkyl in which aryl represents 2- or 3-thienyl, 2-, 3-, or 4-pyridyl, phenyl, or phenyl

monosubstituted by halogen, trifluoromethyl, lower alkoxy, lower alkyl or by a substituent -W-Z in which W represents a direct bond, $C_1$-$C_4$-alkylene, thio-$C_1$-$C_3$-alkylene or oxy-$C_1$-$C_3$-alkylene and Z represents cyano, carboxy, lower alkoxycarbonyl, carbamoyl, N-mono- or N,N-di-lower alkylcarbamoyl; $R^4$ represents $C_1$-$C_4$-alkyl, cyclopropyl or hydroxy-$C_2$-$C_4$-alkyl; $R^5$ and $R^6$ represent hydrogen, lower alkanoyl, lower alkoxy-lower alkanoyl, aroyl, carbamoyl, mono- or di-lower alkylcarbamoyl; and pharmaceutically acceptable salts thereof.

A subembodiment relates to compounds of formula II wherein all residues have meaning as defined hereinbefore except that R is different from $C_1$-$C_4$-alkyl and $R^1$ is different from ($C_5$- or $C_6$)-cycloalkyl-$C_1$-$C_4$-alkyl and 2- or 3-thienyl.

Particularly preferred are said compounds of formula II wherein R represents $C_1$-$C_3$-alkyl or hydrogen; $R^1$ represents $-CH_2CH_2$-(cyclohexyl or cyclopentyl); or $R^1$ represents $-CH_2CH_2$-aryl in which aryl represents 2- or 3-pyridyl, phenyl, or phenyl monosubstituted by a substituent $-CH_2CH_2-Z$ or $-OCH_2-Z$ in which Z represents cyano, carboxy, lower alkoxycarbonyl, carbamoyl, N-mono- or N,N-di-lower alkylcarbamoyl; $R^4$ represents ethyl or hydroxyethyl; $R^5$ and $R^6$ represent hydrogen, lower alkanoyl or lower alkoxy-$C_2$-$C_4$-alkanoyl; and pharmaceutically acceptable salts thereof.

A subembodiment relates to compounds of formula II being disclosed hereinbefore as being particularly preferred wherein all residues have meaning as defined hereinbefore except that R is different from $C_1$-$C_3$-alkyl and $R^1$ is different from $-CH_2CH_2$-(cyclohexyl or cyclopentyl).

Most preferred are the compounds of formula II wherein R represents hydrogen or methyl; $R^1$ represents cyclohexylethyl; or $R^1$ represents 2-phenylethyl, 2-(2-pyridyl)ethyl or 2-phenylethyl substituted in the para position by $-CH_2CH_2Z$ in which Z represents carboxy, lower alkoxycarbonyl, carbamoyl or mono-lower alkylcarbamoyl; $R^4$ represents ethyl; $R^5$ and $R^6$ represent hydrogen; and pharmaceutically acceptable salts thereof.

A subembodiment relates to compounds of formula II being disclosed hereinbefore as being most preferred wherein all residues have meaning as defined hereinbefore except that R is different from methyl and $R^1$ is different from cyclohexylethyl.

A particular preferred embodiment of the invention is also represented by the compounds of formula IIa

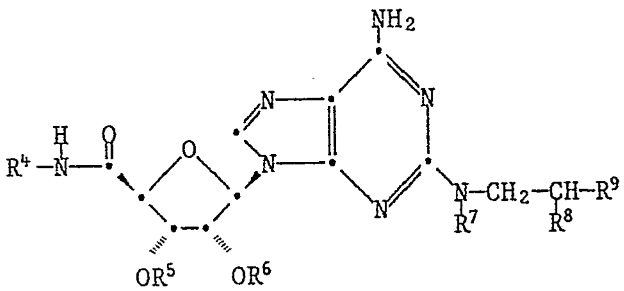

(IIa)

wherein $R^4$ represents ethyl; $R^5$ and $R^6$ represent hydrogen or lower alkanoyl; $R^7$ represents hydrogen or methyl; $R^8$ represents hydrogen or methyl; $R^9$ represents cyclohexyl, phenyl, or phenyl monosubstituted by halogen, trifluoromethyl, lower alkyl, lower alkoxy or $-CH_2CH_2-Z$ in which Z represents carboxy or lower alkoxycarbonyl; and pharmaceutically acceptable salts thereof.

The general definitions used herein have the following meaning within the scope of the present invention.

The term "lower" referred to above and hereinafter in connection with organic radicals or compuonds respectively defines such with up to and including 7, preferably up to and including 4 and advantageously one or two carbon atoms.

Lower alkyl preferably contains 1 to 4 carbon atoms, and represents for example ethyl, propyl, isopropyl, butyl, and advantageously methyl.

Lower alkoxy preferably contains 1 to 4 carbon atoms, and represents for example methoxy, ethoxy, propoxy or isopropoxy.

Lower alkylene preferably contains 1 to 4 carbon atoms, and represents for example methylene, or ethylene.

Lower alkenylene preferably contains 2 to 4 carbon atoms, and represents for example ethenylene, 1- or 2-propenylene.

Halogen is preferably chloro, but may also be fluoro, bromo or iodo.

Cycloalkyl represents preferably 3 to 6 ring membered cycloalkyl, i.e. $C_3$-$C_6$-cycloakyl, which represents cyclopropyl, cyclobutyl, cyclopentyl or cyclohexyl, preferably cyclopropyl.

Cycloalkyl-lower alkyl represents preferably (cyclopentyl or cyclohexyl)-$C_1$-$C_4$-alkyl, advantageously 1- or 2-(cyclopentyl or cyclohexyl)ethyl, propyl or butyl.

Aryl has been defined hereinabove and represents advantageously phenyl or phenyl substituted as described above. Aryl-lower alkyl represents preferably aryl-$C_1$-$C_4$-alkyl in which aryl represents a carbocyclic or heterocyclic aromatic radical as defined above, e.g. benzyl or 1- or 2-phenyl-(ethyl, propyl or butyl), each unsubstituted or substituted on phenyl as defined under aryl above; or 2-, 3- or 4-pyridylmethyl or 2-(2-, 3- or 4-pyridyl)-(ethyl, propyl or butyl); or 1- or 2-naphthylmethyl or 2-(1- or 2-naphthyl)-(ethyl, propyl or butyl). Diaryl-lower alkyl represents preferably diphenyl-$C_1$-$C_4$-alkyl, e.g. omega-diphenyl-(methyl, ethyl or propyl).

Hydroxy-lower alkyl represents preferably 2-, 3- or 4-hydroxy-$C_2$-$C_4$-alkyl, advantageously hydroxyethyl.

Pyridyl represents 2-, 3- or 4-pyridyl, advantageously 2- or 3-pyridyl. Thienyl represents 2- or 3-thienyl.

Aryl-cycloalkyl represents preferably phenyl-$C_3$-$C_6$-cycloalkyl, for example 2-phenylcyclohexyl or 2-phenylcyclopropyl.

A bicyclic benzo-fused 5- or 6-memberd saturated carbocyclic radical, as a substituent $R^1$ depicted by formula B above in which A represents methylene, represents preferably 1,2,3,4-tetrahydro-2-naphthyl or 2-indanyl, each unsubstituted or substituted on benzo portion as indicated above for phenyl under aryl.

A bicyclic benzo-fused 5- or 6-membered saturated heterocyclic radical, as a substituent depicted by formula B above in which A represents oxygen or sulfur, represents preferably 3,4-dihydro-2H-[1]-3-benzopyranyl or 3,4-dihydro-2H-[1]-3-benzothiopyranyl, each unsubstituted or substituted on the benzo portion as indicated above for phenyl under aryl.

Lower alkoxycarbonyl preferably contains 1-4 carbon atoms in the alkoxy portion and represents for example methoxycarbonyl, propoxycarbonyl, isopropoxycarbonyl or advantageously ethoxycarbonyl.

Lower alkanoyl represents preferably $C_1$-$C_4$-alkanoyl, e.eg. acetyl, isobutyryl, or pivaloyl.

Lower alkoxy-lower alkanoyl represents preferably lower alkoxy-$C_2$-$C_4$-alkanoyl, e.g. methoxyacetyl, or 3-ethoxypropionyl.

Aroyl represents preferably benzoyl, benzoyl substituted by one to three of lower alkyl, lower alkoxy, halogen or trifluoromethyl; 2-, 3- or 4-pyridylcarbonyl; or 2- or 3-thienylcarbonyl.

Mono- and di-lower alkylcarbamoyl represents for example N-methyl-, N-ethyl-, N,N-dimethyl- and N,N-diethylcarbamoyl.

Carboxy esterified in form of a pharmaceutically acceptable ester represents advantageously an ester that may be convertible by solvolysis or under physiological conditions to the free carboxylic acid, e.g. lower alkoxycarbonyl; (amino, mono- or di-lower alkyl-amino)substituted lower alkoxycarbonyl; carboxy substituted lower alkoxycarbonyl, e.g. alpha-carboxy-substituted lower alkoxycarbonyl; lower alkoxycarbonyl-substituted lower alkoxycarbonyl, e.g. alpha-lower alkoxycarbonyl-substituted lower alkoxycarbonyl; aryl-substituted lower alkoxycarbonyl, e.g. optionally substituted benzyloxy carbonyl or pyridylmethoxycarbonyl; (hydroxy, lower alkanoyloxy or lower alkoxy)-substituted lower alkoxy carbonyl, e.g. pivaloyloxymethoxycarbonyl; (hydroxy, lower alkanoyloxy or lower alkoxy)-substituted lower alkoxymethoxycarbonyl; bicycloalkoxycarbonyl-substituted lower alkoxycarbonyl, e.g. bicyclo[2,2,1]heptyloxycarbonyl-substituted lower alkoxycarbonyl, especially bicyclo[2,2,1]-heptyloxycarbonyl-substituted methoxycarbonyl such as bornyloxycarbonylmethoxycarbonyl; 3-phthalidoxycarbonyl; (lower alkyl, lower alkoxy, halo)-substituted 3-phthalidoxycarbonyl; lower alkoxycarbonyloxy-lower alkoxycarbonyl, e.g. 1-(methoxy- or ethoxycarbonyloxy)-ethoxycarbonyl; aryloxycarbonyl, e.g. phenoxycarbonyl or phenoxycarbonyl advantageously substituted at the ortho position by carboxy or lower alkoxycarbonyl.

Carboxy derivatized in form of a pharmaceutically acceptable amide represents preferably carbamoyl, mono-lower alkylcarbamoyl or di-lower alkylcarbamoyl.

The pharmaceutically acceptable ester derivatives in which one or more free hydroxy groups are esterified in the form of a pharmaceutically acceptable ester are particularly prodrug esters that may be convertible by solvolysis under physiological conditions to the compounds of formula I having free hydroxy groups.

Preferred as said prodrug pharmaceutically acceptable esters are lower alkanoic acid esters, e.g., the acetic, isobutyric, pivaloic acid esters; lower alkoxy-lower alkanoic acid esters, e.g., the methoxyacetic, 3-ethoxypropionic acid esters; arylcarboxylic acid esters, e.g., the benzoic, nicotinic acid esters; carbamic and mono or di-lower alkylcarbamic acid esters (carbamates), e.g. the mono-or di-ethylcarbamic or N-mono- or di-methylcarbamic acid esters. Most preferred are the lower alkanoic acid and lower alkoxyalkanoic acid esters.

Pharmaceutically acceptable salts are generally acid addition salts, which are preferably such of therapeutically acceptable inorganic or organic acids, such as strong mineral acids, for example hydrohalic, e.g. hydrochloric or hydrobromic acid; sulfuric, phosphoric or nitric acid; aliphatic or aromatic carboxylic or sulfonic acids, e.g. formic, acetic, propionic, succinic, glycollic, lactic, malic, tartaric, gluconic, citric, maleic, fumaric, pyruvic, phenylacetic, benzoic, 4-aminobenzoic, anthranilic, 4-hydroxybenzoic, salicylic, 4-aminosalicylic, pamoic, nicotinic, methanesulfonic, ethanesulfonic, hydroxyethanesulfonic, benzenesulfonic, p-toluenesulfonic, naphthalenesulfonic, sulfanilic, cyclohexylsulfamic acid; or ascorbic acid.

For compounds having a free carboxy group, pharmaceutically acceptable salts are also derived from bases, e.g. alkali metal salts, such as the sodium salt, or salts derived from pharmaceutically acceptable amines, such as tromethamine.

The novel compounds of the invention are active in state of the art in vitro and in vivo test systems, indicative of adenosine receptor agonist activity in mammals.

The adenosine receptor agonists of the invention are useful in mammals including man for the treatment of central nervous system disorders, particularly psychoses such as schizophrenia, and of cardiovascular disorders, particularly hypertension and thrombosis.

The above-cited properties are demonstrable in in vitro and in vivo tests, using advantageously mammals, e.g. rats, dogs, monkeys or isolated organs, tissues and preparations thereof. Said compounds can be applied in vitro in the form of solutions, e.g. preferably aqueous solutions, and in vivo either enterally or parenterally advantageously orally or intravenously, e.g. within gelatin capsules, as starch suspensions or in aqueous solutions. The dosage in vitro may range between about $10^{-5}$ molar and $10^{-9}$ molar concentrations. The dosage

in vivo may range between about 0.001 and 25 mg/kg/day, preferably between about 0.0025 and 10 mg/kg/day depending on the compound and the route of administration.

Adenosine-2 (A-2) receptor binding properties, indicative of the adenosine-2 receptor agonist activity of the compounds of the invention are determined in vitro by determining their ability to inhibit the specific binding of $^3H$-5′-N-ethylcarboxamidoadenosine ($^3H$-NECA), e.g. essentially as described by R. F. Bruns et al., Mol. Pharmacol. 29, 331 (1986), in striatal membrane preparations from corpus striatum of male Sprague-Dawley rats. The concentration of a particular compound required to displace the specific binding of 4 nM $^3H$-NECA is determined in the presence of 50 nM cyclopentyladenosine.

Adenosine-1 (A-1) receptor binding properties of the compounds of the invention indicative of adenosine-1-receptor agonist activity are determined, e.g., essentially according to R.F. Bruns et al. in Proc. Natl. Acad. Sci. U.S.A. 77, 5547 (1980), by determining their ability to inhibit the specific binding of $^3H$-cyclohexyl-adenosine ($^3H$-CHA) in rat brain membrane preparations from male Srague-Dawley rats. The concentration of a particular compound required to displace the specific binding of 1 nM $^3H$-CHA is determined.

Selectivity for the adenosine-2 (A-2) receptor can be ascertained by compring the relative potency in the two adenosine receptor assays.

Indicative of in vivo adenosine receptor agonist activity, the hypotensive activity of the compounds of the invention as well as their effect on heart rate can be measured in normotensive or spontaneous hypertensive rats on intravenous or oral administration.

Typically, the blood pressure lowering effect in normotensive rats can be determined as follows.

Adult male rats weighing 300-400 g are anesthetized using Inactin (100 mg/kg, i.p.). A femoral artery and contralateral vein are cnnulated for direct blood pressure measurement and i.v. drug administration, respectively. Animals are allowed a 15 minute equilibration period before testing. Vehicle (1 ml/kg, i.v.) is administered over a 30 second period followed by a 0.3 ml saline flush administered over a 30 second period. Changes in diastolic blood pressure are recorded using a Beckman polygraph while heart rate is recorded as a derivative of the blood pressure pulse. The test compound is administered in the same manner as vehicle and a dose response curve is established. Percent changes in heart rate and blood pressure are recorded.

The blood pressure lowering effect in the spontaneous hypertensive rat is determined on oral administration.

Antipsychotic activity can be demonstrated e.g. by measuring the inhibition of one-way conditioned avoidance or Sidman avoidance in the rat, or by measuring the anatogonism of the behavioral stimulant effects of apomorphine.

Antithrombotic activity can be demonstrated by measuring the inhibition of collagen-induced platelet aggregation.

The compounds of the invention which are selective as adenosine-2 receptor agonists effectively lower blood pressure without any significant effect on the heart rate.

Most selective as adenosine-2 receptor agonists and preferred are the compounds of formula II and IIa as defined above.

Illustrative compounds of the invention have an $IC_{50}$ of about $2 \times 10^{-8}$M in the in vitro adenosine-2 receptor binding assay, and lower blood pressure at a dose as low as about 0.005 mg/kg i.v. in the normotensive rat, or about 3 mg/kg p.o. on the spontaneous hypertensive rat. Said comounds are about 100 fold more selective for the A-2 than for the A-1 receptor in vitro.

The compounds of the invention, i.e. of formula I and hereincited derivatves thereof, may be prepared by process

a) which comprises condensing a compound of the formula III

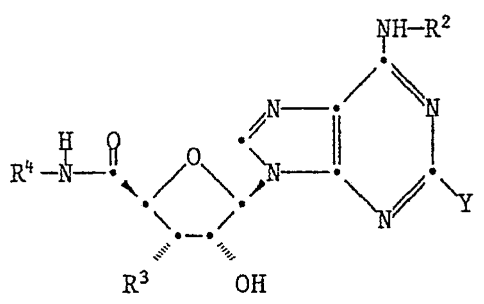

(III)

wherein $R^2$, $R^3$ and $R^4$ have meaning as defined above and Y represents a leaving group, with a compound of the formula IV

$R^1$-NH-R (IV)

wherein R and $R^1$ have meaning as defined above; and, if required, temporarily protecting any interfering reactive group(s) in the starting materials and then subsequently removing the protecting groups to yield a resulting compound of formula I; or by process

b) which comprises condensing a compound of the formula V

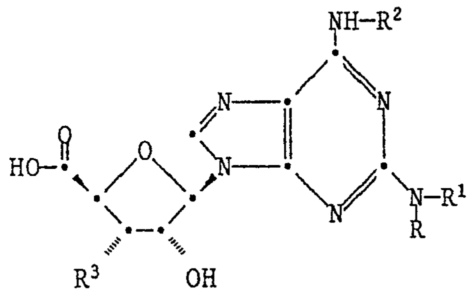

(V)

wherein $R^1$, $R^2$ and $R^3$ having meaning as defined above, or a reactive functional derivative thereof, with an amine of the formula VI

$R^4$-$NH_2$ (VI)

wherein $R^4$ has meaning as defined above; and, if required, temporarily protecting any interfering reacting group(s) in the starting materials and then subsequently removing the protecting groups to yield a resulting compound of formula I; and in any of these processes, if desired, converting a resulting compound of formula I into another compound of the invention, and if desired, converting a resulting free compound into a salt or a resulting salt into a free compound or into another salt, and if required, separating any mixture of isomers or racemates obtained into the single isomers or racemates, and if required, resolving a racemate into the optical antipodes. Of these processes process a) is preferred.

A leaving group in the above processes represents reactive esterified or etherfied hydroxy and is e.g. especially halo, for example chloro, bromo or iodo, aliphatically or aromatically substituted sulfonyloxy, for example methylsulfonyloxy or 4-methylphenylsulfonyloxy (tosyloxy), or aliphatically substituted thio, for example lower alkylthio such as methylthio.

In the processes cited herein, reactive functional derivatives of carboxylic acids represent, for example, anhydrides especially mixed anhydrides, acid halides, acid azides, lower alkyl esters and activated esters thereof. Mixed anhydrides are preferably such from pivalic acid, or a lower alkyl (ethyl, isobutyl) hemiester of carbonic acid; acid halides are for example chlorides or bromides; activated esters are for example succinimido, phthalimido or 4-nitrophenyl esters; lower alkyl esters are for example the methyl or ethyl esters.

In starting compounds and intermediates which are converted to the compounds of the invention in a manner described herein, functional groups present, such as amino and hydroxy, are optionally protected by conventional protecting groups that are common in preparative organic chemistry. Protected amino and hydroxy groups are those that can be converted under mild conditions into free amino and hydroxy groups without the molecular framework being destroyed or undesired side reactions taking place.

Well-known protecting groups that meet these conditions and their introduction and removal are described, for example, in J.F.W. McOmie, "Protective Groups in Organic Chemistry", Plenum Press, London, New York 1973, T.W. Greene, and "Protective Groups in Organic Synthesis", Wiley, New York 1984.

For example, a hydroxy group may be protected in the form of esters, e.g. as acyl derivatives such as the lower alkanoyl, benzoyl, benzyloxycarbonyl or lower alkoxycarbonyl esters, or such hydroxy group may be protected in the form of ethers, e.g. as the lower alkyl, 2-tetrahydropyranyl, trityl or benzyl ethers.

Hydroxy groups on adjacent carbon atoms can also be protected e.g. in the form of ketals or acetals, such as lower alkylidene e.g. isopropylidene, benzylidene or 5- or 6-membered cycloalkylidene e.g. cylcopentylidene or cyclohexylidene derivatives.

In a resulting protected compound of formula I or intermediate, in which one or more of the functional groups are protected, the protected functional groups, e.g. hydroxy groups, can be liberated in manner known per se, e.g. by means of solvolysis, especially hydrolysis with acid, or by hydrogenolysis.

A compound of the invention with both 2′ and 3′-hydroxy groups, e.g. a compound of formula I or Ia wherein $R^3$ represents hydroxy and wherein both of the adjacent 2′- and 3′-hydroxy groups are protected in the form of ether, acetal or ketal derivatives as described above, e.g. as the isopropylidene (acetonide) derivative, can be converted to a compound of formula I or Ia wherein $R^3$ represents hydrogen by elimination of the 3′-substituent by treatment with a strong base, e.g. sodium hydride in anhydrous isopropanol (sodium isopropoxide) to first yield a compound with a double bond between the 3′-4′-carbon atoms, said double bond being subsequently reduced, e.g. by catalytic hydrogenation.

The preparation of the compounds of the invention according to process a) which involves the displacement of a leaving group Y (e.g. chloro) in a compound of the formula III or a protected derivative by an amine of the formula IV is preferably carried out at elevated temperature, e.g. at a temperature ranging from about 75 to 150° C, with an excess of the amine, in the absence or presence of a solvent, particularly a polar solvent such as dimethylformamide, or under elevated pressure, or in the presence of a base such as triethylamine or potassium carbonate.

The starting materials of formula III can be prepared by condensing a compound of the formula VII

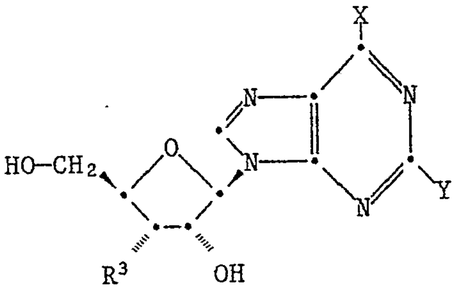

(VII)

or a compound of formula VII in partially protected from, wherein X and Y represent a leaving group, and $R^3$ has meaning as defined above, with a compound of the formula VIII

$R^2$-$NH_2$ (VIII)

wherein $R^2$ has meaning as defined above; oxidizing the primary alcohol group in a resulting compound of formula IX

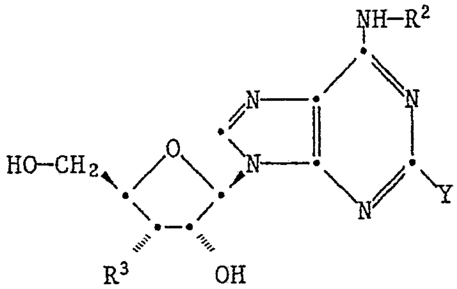

(IX)

in which any sceondary hydroxy groups are in protected form, and wherein Y, $R^2$ and $R^3$ have meaning as defined above, to the corresponding carboxylic acid; and converting said carboxylic acid to a coresonding amide of formula III.

The intermediates of formula VII, and protected derivatives thereof, e.g. in which X and Y represent halogen, particularly chloro, are known or are prepared according to methods known in the art relating to N-(β-D-ribofuranosyl)-purine derivatives, for example as described in Chem. Pharm. Bull. 23, 758 (1975).

The displacement of the leaving group X in a compound of formula VII with an amine of formula VIII is carried out essentially as described above for process a), preferably using about one mole equivalent of the amine, so as to minimize the displacement of the less reactive leaving group Y.

The oxidation of the resulting e.g. 2-halo substituted adenosine derivatives, in which secondary hydroxy groups are in protected form is carried out for example with potassium permanganate as described in U.S. patent 4,167,565.

The resulting carboxylic acid is then first converted to a reactive derivative thereof, e.g. the acid chloride, which is condensed with an amine of the formula VI under condition well-known in the art, e.g. as described in U.S. patent 4,167,565.

The starting materials of formula IV, VI and VIII are either known in the art, or are prepared using methods known in the art, and as described herein.

The preparation of the compounds of the invention according to process b) involving the conversion of an acid of formula V to a compound of formula I can be carried out using methodology as described above.

The compounds of the invention or intermediates leading thereto can be converted into other compounds of the invention or corresponding intemediates using chemical methodology known in the art and as illustrated herein.

The conversion of compounds of formula I containing free hydroxy groups ester derivatives thereof may be carried out by condensation with a corresponding carboxylic acid, advantageously as a reactive functional derivative thereof, according to acylation (esterification) procedures well-known in the art.

The conversion of the compounds of formula I into pharmaceutically acceptable esters, wherein the 2′-hydroxy group (and 3′-hydroxy group if present) is esterified, can be carried out by condensation with a corresponding carboxylic acid or reactive derivative thereof, according to esterification procedures known in the art relating to nucleoside chemistry. For example, an appropriate carboxylic acid anhydride such as acetic anhydride is condensed with a compound of formula I in the presence of a suitable base, e.g. pyridine, triethylamine, 4-(dimethylamino)-pyridine, in an inert solvent such as acetonitrile.

A compound of formula I containing a primary amino group (e.g. wherein $NRR^1$ or $NHR^2$ = $NH_2$) may be converted to a compound of formula I wherein $NRR^1$ or $NHR^2$ represents a secondary amino group, e.g. wherein $R^1$ or $R^2$ represents e.g. aryl-lower alkyl, by treatment with a reactive derivative of the alcohol corresponding to $R^1$ or $R^2$, e.g. with an aryl-lower alkyl halide such as an aryl-lower alkyl iodide, according to

methodology well-known in the art for alkylation of amines. Similarly, a secondary amine wherein R represents hydrogen may be converted to a tertiary amine wherein R represents lower alkyl.

The above-mentioned reactions are carried out according to standard methods, in the presence or absence of diluents, preferably such as are inert to the reagents and are solvents thereof, of catalysts, condensing or said other agents respectively and/or inert atmospheres, at low temperatures, room temperature or elevated temperatures preferably near the boiling point for the solvents used, and at atmospheric or superatmospheric pressure.

The invention further includes any variant of the present processes, in which an intermediate product obtainable at any stage thereof is used as starting material and the remaining steps are carried out, or in which the starting materials are formed under the reaction conditions, or in which the reaction components are used in the form of their salts or optically pure antipodes. Whenever desirable, the above processes are carried out after first suitably protecting any potentially interfering reactive functional groups, as illustrated herein.

Advantageously, those starting materials should be used in said reactions that lead to the formation of those compounds indicated above as being preferred.

The invention also relates to novel starting materials and processes for their manufacture.

Depending on the choice of starting materials and methods, the new compounds may be in the form of isomers, for example, as diasteromers, as optical isomers (antipodes), as racemates, or as mixtures thereof.

In case diastereomeric mixtures of the above compounds or intermediates are obtained, these can be separated into the single racemic or optically active isomers by methods in themselves known, e.g. by fractional distillation, crystallization or chromatography.

Any racemic products of formula I or basic intermediates can be resolved into the optical antipodes, for example, by separation of diastereomeric salts thereof, e.g., by the fractional crystallization of d- or $\ell$-(tartrate, dibenzoyltartrate, mandelate or camphorsulfonate) salts.

Advantageously, the more active of the antipodes of the compounds of this invention is isolated.

Finally, the compounds of the invention are either obtained in the free form, or as a salt thereof. For example, any resulting free base can be converted into a corresponding acid addition salt, preferably with the use of a pharmaceutically acceptable acid or anion exchange preparation, or resulting salts can be converted into the corresponding free bases, for example, with the use of a stronger base, such as a metal or ammonium hydroxide, or any basic salt, e.g., an alkali metal hydroxide or carbonate, or a cation exchange preparation. These or other salts, for example, the picrates, can also be used for purification of the bases obtained; the bases are then first converted into salts. In view of the close relationship between the free compounds and the compounds in the form of their salts, whenever a compound is referred to in this context, a corresponding salt is also intended, provided such is possible or appropriate under the circumstances.

The compounds, including their salts, may also be obtained in the form of their hydrates or include other solvents used for the crystallization.

The present invention also relates to the use of the compounds of the invention for the preparation of pharmaceutical compositions, especially pharmaceutical compositions having selective adenosine-2-receptor stimulating activity which can be used for the treatment of e.g. phsychotic conditions, such as schizophrenia, and cardiovascular conditions, such as hypertension, thrombosis and atherosclerosis.

The pharmaceutical compositions according to the invention are those suitable for enteral, such as oral or rectal, transdermal and parenteral adminstration to mammals, including man, for the treatment of diseases responsive to adenosine-2 receptor stimulation as given above, such as hypertension, comprising an effective adenosine-2 receptor stimulating amount of a compound of the invention, alone or in combination with one or more pharmaceutically acceptable carriers.

The pharmacologically active compounds of the invention are incorporated into pharmaceutical compositions comprising an effective amount thereof in conjunction or admixture with excipients or carriers suitable for either enteral or parenteral application. Preferred are tablets and gelatin capsules comprising the active ingredient together with a) diluents, e.g. lactose, dextrose, sucrose, mannitol, sorbitol, cellulose and/or glycine; b) lubricants, e.g. silica, talcum, stearic acid, its magnesium or calcium salts and/or polyethylene glycol; for tablets also c) binders, e.g. magnesium aluminum silicate, starch paste, gelatin, tragacanth, methylcellulose, sodium carboxymethylcellulose and/or polyvinylpyrrolidone; if desired, d) disintegrants, e.g. starches, agar, alginic acid or its sodium salt, or effervescent mixtures; and/or e) absorbents, colorants, flavors and sweeteners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, the compositions may also contain other therapeutically valuable substances. Said compositions are prepared according to conventional mixing, granulating or coating methods, respectively, and contain about 0.1 to 75 %, preferably about 1 to 50 %, of the active ingredient.

Suitable formulations for transdermal application include an effective amount of a compound of the invention with carrier. Advantageous carriers include absorbable pharmacologically acceptable solvents to assist passage through the skin of the host. Characteristically, transdermal devices are in the form of a bandage comprising a backing member, a reservoir containing the compound, optionally with carriers, optionally a rate controlling barrier to deliver the compound to the skin of the host at a controlled and predetermined rate over a prolonged period of time, and means to secure the device to the skin.

The present invention also relates to the use of compounds of the invention having adenosine-2 receptor stimulating properties and pharmaceutical compositions comprising said compounds for the treatment in mammals of disorders responsive to selective adenosine receptor stimulation, particularly psychotic conditions (e.g. schizophrenia) and cardiovascular conditions (e.g. hypertension and thrombosis).

One aspect relates advantageously to the method of treatment of cardiovascular disorders in mammals, e.g. such responsive to adenosine-2 receptor stimulation, for example hypertension, using an effective amount of a compound of the invention, preferably in the form of above-cited pharmaceutical compositions.

The dosage of active compound administered is dependent on the species of warm-blooded animal (mammal), the body weight, age and individual condition, and on the form of administration.

A unit dosage for a mammal of about 50 to 70 kg may contain between about 5 and 100 mg of the active ingredient.

The following examples are intended to illustrate the invention and are not to be construed as being limitations thereon. Temperatures are given in degrees Centigrade. If not mentioned otherwise, all evaporations are performed under reduced pressure, preferably between about 2 and 13 kPa. The structure of final products, intermediates and starting materials is confirmed by analytical methods, e.g. microanalysis and spectroscopic characteristics (e.g. MS, IR, NMR).

The numbering of the positions of the adenine or purine ring system is as conventionally used in the art (e.g. Merck Index, tenth edition).

Example 1:

A mixture of 1.15 g of 2-(2-phenethylamino)-2′,3′-O-isopropylidene-adenosine-5′-(N-ethyl)-carboxamide and 25 ml of IN hydrochloric acid is heated at 65°C for 1 hour. The reaction mixture is neutralized and the product is extracted with ethyl acetate.

After drying over magnesium sulfate, the solvent is removed in vacuo and the residue it triturated with ether to afford 2-(2-phenethylamino)-adenosine-5′-(N-ethyl)-carboxamide, m.p. 115-118°C.

The starting material is prepared as follows: A mixture of 1.04 g of 2-chloro-2′,3′-O-isopropylidene-adenosine-5′-(N-ethyl)-carboxamide (U.S. Patent 4,167,565) and 8 g of 2-phenethylamine is heated at 130°C for 2 hours. After cooling the excess 2-phenethylamine is removed in vacuo and the residue is chromotographed on silica gel with 5% methanol in dichloromethane as the eluent to afford 2-(2-phenethylamino)-2′,3′-O-isopropylidene-adenosine-5′(N-ethyl)-carboxamide.

Example 2:

Prepared in a similar manner are:

a) 2-(2-phenethylamino)-adenosine-5′-(N-cyclopropyl)-carboxamide, m.p. 115-118°C;

b) 2-(p-methoxy-2-phenethylamino)-adenosine-5′-(N-ethyl)-carboxamide, m.p. 110-115°C;

c) 2-(p-chloro-2-phenethylamino)-adenosine-5′-(N-ethyl)-carboxamide, m.p. 110-115°C;

d) 2-(2-phenethylamino)-adenosine-5′-(N-methyl)-carboxamide, m.p. 188-190°C;

e) 2-(2-phenethylamino)-adenosine-5′-(N-2-hydroxyethyl)-carboxamide, m.p. 157-160°C;

f) 2-(p-fluoro-2-phenethylamino)-adenosine-5′(N-ethyl)-carboxamide, m.p. 108-112°C;

g) 2-[p-(2-carboxyethyl)-2-phenethylamino]-adenosine-5′-(N-ethyl)-carboxamide, m.p. 197-202°C; hydrochloride salt, m.p. 200-203°C; tromethamine salt, m.p. 100°C; sodium salt, m.p. 160-165°C.

The amine starting material is prepared as follows: A mixture of 5 g of p-bromophenylacetonitrile, 4.6 ml of t-butyl acrylate, 57 mg of palladium diacetate, 310 mg of tri-o-tolylphosphine and 12 ml of triethylamine is refluxed for 5 hours. The reaction mixture is diluted with ethyl acetate and washed with 10 % hydrochloric acid and saturated sodium bicarbonate solution. After drying over magnesium sulfate the solvent is removed in vacuo to yield t-butyl p-(cyanomethyl)-phenylacrylate. This material is dissolved in ethanol and hydrogenated over 1.1 g of 10 % palladium on carbon catalyst for 3 days at 300 kPa pressure of hydrogen. After filtration the solvent is removed in vacuo and the residue is chromatographed on silica gel with ether/hexane (1:1) as the eluent to afford p-(2-t-butoxycarbonyl-ethyl)-phenylacetonitrile; 2.8 g of this material is dissolved in 90 ml of tetrahydrofuran and 50 ml of methanol and to this is added 6.2 g of cobalt chloride in 90 ml of water followed by 2.1 g of sodium borohydride in small portions. After filtration and removal of solvent, the residue is chromatographed on silica gel with 7.5 % ammonia saturated methanol in dichloromethane as the eluent to afford p-(2-t-butoxycarbonyl-ethyl)-2-phenethylamine as an oil.

h) 2-[p-(2-carboxyethenyl)-2-phenethylamino]-adenosine-5′-(N-ethyl)-carboxamide hydrochloride, m.p. 178-181°C.

The starting material is prepared by reduction of t-butyl p-(cyanomethyl)-phenylacrylate (as obtained in example 2 g) to p-t-butoxycarbonyl-ethenyl)-2-phenethylamine with sodium borohydride and cobalt chloride as described under g) above.

i) 2-[p-(2-carboxyethenyl)-2-phenethylamino]-adenosine-5′-(N-ethyl)-carboxamide hydrochloride, m.p. 137-141°C.

The amine starting material is prepared as follows: A mixture of 3 g of p-hydroxyphenylacetonitrile, 3.6 ml of t-butyl bromoacetate, 6.5 g of potassium carbonate in 45 ml of dimethylformamide is stirred at room temperature for 16 hours. After dilution with water the product is extracted with ether. The ethyl layer is washed with 1N sodium hydroxide, dried over magnesium sulfate and the solvent removed in vacuo to yield p-(t-butoxycarbonylmethoxy)-phenylacetonitrile which is reduced to p-(t-butoxycarbonylmethoxy)-2-phenethylamine with sodium borohydride/cobalt chloride as described for the starting material under g).

j) 2-(S-2-phenylpropylamino)-adenosine-5′-(N-ethyl)-carboxamide, m.p. 117-121° C, prepared from the levorotatory (S)-2-phenylpropylamine, J. Med. Chem. 17, 717 (1974);

k) 2-(N-methyl-2-phenethylamino)-adenosine-5′-(N-ethyl)-carboxamide hydrochloride, m.p. 115-119° C, prepared from N-methylphenethylamine;

l) 2-(p-carboxymethyl-2-phenethylamino)-adenosine-5′-(N-ethyl)-carboxamide hydrochloride, m.p. 140-145° C.

The amine starting material for compound l) is prepared as follows: A mixture of 20 g of p-bromophenylacetic acid, 30 ml of ether, 1 ml of sulfuric acid and 35 ml of isobutylene is shaken in a sealed bottle for 24 hours. The reaction mixture is diluted with ether and washed with sodium hydroxide solution. After drying over magnesium sulfate the ether is removed in vacuo to afford the t-butyl ester as an oil. A mixture of 9.6 g of this material is refluxed with a mixture of 6.1 g of N-vinylphthalimide, 160 mg of palladium acetate, 800 mg of tri-o-tolyl-phosine, 100 ml of acetonitrile and 8 ml diisopropylethylamine for 24 hours. The reaction is diluted with water, the resulting precipitate is collected and recrystallized from methanol/dichloromethane. The resulting solid is hydrogenated at 400 kPa pressure over 2 g of 10 % palladium on carbon catalyst in 100 ml of ethanol and 100 ml of tetrahydrofuran for 16 hours at room temperature. After removal of the solvent in vacuo the residue is heated at reflux with 10 ml of hydrazine hydrate and 20 ml of ethanol for 2 hours. The reaction is diluted with ether and washed with 5 % potassium hydroxide solution. The ether is dried over magnesium sulfate solution and the solvent is removed in vacuo. The residue is chromotographed on silica gel, with 5 % ammonia saturated methanol in dichloromethane as the eluent, to afford p-(t-butoxycarbonylmethyl)-2-phenethylamine as an oil.

m) 2-[p-(dimethylaminocarbonylmethyl)-2-phenethylamino]-adenosine-5′-(N-ethyl)-carboxamide.

The amine starting material is prepared as follows: A mixture of 6 g of p-bromophenylacetic acid in 100 ml of dichloromethane and 5 ml of oxalyl chloride is stirred at room temperature for 16 hours. After removal of the solvent in vacuo the residue is dissolved in dichloromethane and treated with excess dimethylamine at room temperature. After 1 hour the reaction mixture is washed with water, the organic layer is dried over magnesium sulfate and the solvent is removed in vacuo to afford p-bromo-N,N-dimethyl-phenylacetamide as an oil, which is converted to p-(dimethylaminocarbonylmethyl)-2-phenethylamine as described for the starting material under l).

n) 2-(2-cyclohexylethyl)-adenosine-5′-(N-ethyl)-carboxamide hydrochloride, m.p. 154-157° C;

o) 2-(2-cyclopentylethyl)-adenosine-5′-(N-ethyl)-carboxamide;

p) 2-(N-methyl-2-cyclohexylethylamino)-adenosine-5′-(N-ethyl)-carboxamide;

q) 2-(p-carboxy-2-phenethylamino)-adenosine-5′-(N-ethyl)-carboxamide. The amine starting material is prepared using methodology under l) from p-bromobenzoic acid.

Example 3:

The following compounds of formula Ia wherein $R^3$ represents hydroxy can be prepared substantially according to the procedures previously described herein.

| Compound | $NRR^1$ | $R^4$ |
|---|---|---|
| a) | 3,4-dihydro-5-methoxy-2H-[1]-benzothio-pyran-3-ylamino | $CH_2CH_3$ |
| b) | 2-indanylamino | $CH_2CH_3$ |
| c) | 1,2,3,4-tetrahydro-2-naphthylamino | $CH_2CH_3$ |
| d) | 3,4-dihydro-2H-[1]-benzopyran-3-ylamino | $CH_2CH_3$ |
| e) | $NH-CH_2CH_2-p-C_6H_4-OCH_2CON(C_2H_5)_2$ | $CH_2CH_3$ |
| f) | 2,2-diphenylethylamino | $CH_2CH_3$ |
| g) | 2-(2-pyridyl)-ethylamino | $CH_2CH_3$ |
| h) | 2-(2-thienyl)-ethylamino | $CH_2CH_3$ |
| i) | (9-9H-fluorenyl)-methylamino | $CH_2CH_3$ |
| j) | N-methyl-2-(2-pyridyl)-ethylamino | $CH_2CH_3$ |
| k) | N-methyl-2-(2-thienyl)-ethylamino | $CH_2CH_3$ |
| l) | 2-(2-pyridyl)-propylamino | $CH_2CH_3$ |

The starting material for compound a) is prepared as follows: To a cooled mixture of 30.6 g of m-methoxybenzenethiol, 54.4 g of 45 % potassium hydroxide in 100 ml of dimethylsulfoxide is added 36.0 g of alpha-(bromomethyl)acrylic acid in 25 ml of dimethylsulfoxide at such a rate as to maintian the reaction temperature at 50-55°C. After 1 hour the reaction mixture is diluted with water and washed with ether. After acidification, the product is extracted with ether, the organic layer is dried over magnesium sulfate and the solvent is removed in vacuo to afford alpha-(3-methoxybenzenethiomethyl)acrylic acid. This material is dissolved in 570 ml of o-dichlorobenzene and 7.2 g of triethylamine and heated to 200°C for 5 hours. After cooling, the products are extracted with sodium bicarbonate solution, the aqueous layer is acidified and the products extracted with ethe. After drying over magnesium sulfate, the solvent is removed in vacuo to afford a mixture of 3,4-dihydro-5-methyl-2H-[1]-benzothiopyran-3-carboxylic acid and 3,4-dihydro-7-methoxy-2H-[1]-benzothiopyran-3-carboxylic acid.

This mixture of acids is dissolved in 500 ml of t-butyl alcohol and treated with 17 g of triethylamine and 36 ml of diphenylphosphoryl azide. After 5 hours reflux, the solvent is removed in vacuo and the residue is dissolved in ether and washed with 1N sodium hydroxide and 1N hydrochloric acid. After drying over magnesium sulfate, the solvent is removed in vacuo and the residue is chromatographed in silica gel (1 kg) with ether/hexane (1:4) as the eluent to afford in succession N-t-butoxycarbonyl-3,4-dihydro-5-methoxy-2H-[1]-benzothiopyran-3-amine and N-t-butoxycarbonyl-3,4-dihydro-7-methoxy-2H-[1]-benzothiopyran-3-amine.

A solution of 10 g of N-t-butoxycarbonyl-3,4-dihydro-5-methoxy-2H-[1]-benzothiopyran-3-amine in 30 ml of trifluoroacetic acid is kept at room temperature for 1 hour. The solvent is removed in vacuo, the residue is treated with 1N sodium hydroxide and the product is extracted with ether. After drying over magnesium sulfate, the solvent is removed in vacuo to afford 3,4-dihydro-5-methoxy-2H-[1]-benzothiopyran-3-amine as an oil.

Example 4:

A mixture of 2.70 g of 2-[p-(2-t-butoxycarbonylethyl)-2-phenethylamino]-2′,3′-O-isopropylidene-adenosine-5′-(N-ethyl)-carboxamide and 45 ml of 1N hydrochloric acid is heated at 65°C for 1 hour. The reaction mixture is cooled, the resulting precipitate is collected, washed first with ice water and then with a mixture of ethyl acetate and ether to yield 2-[p-(2-carboxyethyl)-2-phenethylamino]-adenosine-5′-(N-ethyl)-carboxamide hydrochloride, m.p. 200-203°C (of example 2 g).

The starting material is prepared as follows: A mixture of 2.31 g of t-butyl p-(cyanomethyl)-phenylacrylate (example 2 g), 6.28 g of concentrated aqueous ammonium hydroxide and 0.46 g of 5 % rhodium on alumina in 50 ml of absolute ethanol is hydrogenated at 300 kPa pressure and room temperature for 22 hours. A second portion of 0.46 g of rhodium on alumina is added and hydrogenation is continued for 6 more hours. The reaction mixture is filtered, the catalyst is washed with ethanol, and the filtrate is evaporated to dryness. The residue is dissolved in 50 ml of ethyl acetate, hydrogen chloride gas is bubbled into the solution for 10 minutes and the solution is evaporated to dryness. The product is triturated with ether and collected to yield p-(2-t-butoxycarbonylethyl)-2-phenethylamine hydrochloride which is then converted to the free base.

A mixture of 4.0 of 2-chloro-2′,3′-O-isopropylidene-adenosine-5′-(N-ethyl)-carboxamide and 14.0 g of p-(2-t-butoxycarbonylethyl)-2-phenethylamine is heated at 130°C for 3 hours. The reaction mixture is

dissolved in dichloromethane, the solution is washed with sodium bicarbonate solution and evaporated to dryness. The residue is crystallized from ether to yield 2-[p-(2-t-butoxycarbonylethyl)-2-phenethylamino]-2',3'-O-isopropylidene-adenosine-5'-(N-ethyl)-carboxamide, m.p. 180°C.

Example 5:

A solution of 12 mg of 2β-{2-[p-(2-t-butoxycarbonylethyl)-2-phenethylamino]-9-adenyl}-3-alpha-hydroxy-2,3-dihydrofuran-5-N-ethylcarboxamide in 1.5 ml of ethanol to which is added 10 mg of 5 % rhodium on carbon is hydrogenated at room temperature and 300 kPa pressure for 30 hours. The catalyst is filtered off and the solution is evaporated to dryness to yield a mixture of isomers comprising 2-[p-(2-carboxyethyl)-2-phenethylamino]-3'-deoxyadenosine-5'-(N-ethyl)-carboxamide; NMR ($CD_3OD$): 8.0 (s, 1H), 5.93 (d,1H).

The starting material is prepared as follows: Sodium hydride (6 mg of 60 % dispersion in mineral oil) is added to a solution of 20 mg of 2-[p-(2-t-butoxy-carbonylethyl)-2-phenethylamino]-2',3'-O-isopropylidene-adenosine-5'-(N-ethyl)-carboxamide in 25 ml of anhydrous isopropanol. The reaction mixture is heated at 70°C for 6 hours. The reaction mixture is cooled and the reaction is quenched with 0.5 ml pH 6 phosphate buffer, and the mixture is evaporated to dryness. The resulting product is chromatographed on silica gel eluting with 10 % methanol in dichloromethane to yield 2-{2β-[p-(2-t-butoxycarbonyl ethyl)-2-phenethylamino]-9-adenyl}-3-alpha-hydroxy-2,3-dihydrofuran-5-N-ethylcarboxamide as an oil; NMR ($CD_3OD$): 7.82 (s,1H), 6.36 (d,1H), 6.1 (d,1H), 5.52 (t,1H).

Example 6:

2-(p-(2-Carboxyethyl)-2-phenethylamino)-adenosine-5'-(N-ethyl)carboxamide is treated under reflux with absolute ethanol in the presence of a catalytic amount of concentrated sulfuric acid to yield 2-(p-(2-ethoxycarbonylethyl)-2-phenethylamino)-adenosine-5'-(N-ethyl)-carboxamide.

Example 7:

a) Preparation of 10,000 tablets each containing 10 mg of the active ingredient having a formula as follows:

2-(2-phenethylamino)-adenosine-5'-(N-ethyl)-carboxamide 100.00 g
Lactose 2400.00g
Corn starch 125.00 g
Polyethylene glycol 6000 150.00 g
Magnesium stearate 40.00 g
Purified water q.s.

Procedure:

All the powers are passed through a screen with openings of 0.6 mm. Then the drug substance, lactose, magnesium stearate and half of the starch are mixed in a suitable mixer. The other half of the starch is suspended in 65 ml of water and the suspension added to the boiling solution of the polyethylene glycol in 260 ml of water. The paste formed is added to the powders, which are granulated, if necessary, with an additional amount of water. The granulate is dried overnight at 35°C, broken on a screen with 1.2 mm openings and compressed into tablets, using concave punches uppers bisected.

b) Preparation of 1,000 capsules each containing 10 mg of the active ingredient having a formula as follows:

2-[p-(2-carboxyethyl)-2-phenethylamino]-adenosine-5'-(N-ethyl)-carboxamide hydrochloride 10.0 g
Lactose 207.0 g
Modified starch 80.0 g
Magnesium stearate 3.0 g

Procedure:

All the powders are passed through a screen with openings of 0.6 mm. Then the drug substance is placed in a suitable mixer and mixed first with the magnesium stearate, then with the lactose and starch until homogeneous. No. 2 hard gelatin capsules are filled with 300 mg of said mixture each, using a capsule filling machine.

**Claims**

1. A compound of the formula I

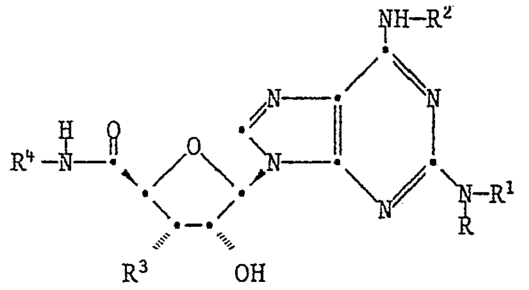

(I)

wherein R represents hydrogen or lower alkyl; $R^1$ represents cycloalkyl, cycloalkyl-lower alkyl, 2-norbornanyl, 2-norbornanyl-lower alkyl, aryl, aryl-lower alkyl, aryl-cycloalkyl, 9-fluorenyl, diaryl-lower alkyl or 9-fluorenyl-lower alkyl; or $R^1$ represents a bicyclic benzo-fused 5- or 6-membered saturated carbocyclic radical of a benzo-fused 5- or 6-membered saturated heterocyclic radical containing a heteroatom selected from oxygen and sulfur which is directly attached to the fused benzene ring, any said bicyclic radical being unsubstituted or substituted on the benzo portion by lower alkyl, lower alkoxy, hydroxy, halogen or trifluoromethyl, or by a substituent -W-Z in which W represents a direct bond, lower alkylene, lower alkenylene, thio-lower alkylene or oxy-lower alkylene and Z represents cyano, carboxy or carboxy derivatized in the form of a pharmaceutically acceptable ester or amide; $R^2$ represents hydrogen, lower alkyl or aryl-lower alkyl; $R^3$ represents hydrogen or hydroxy; $R^4$ represents hydrogen, lower alkyl, aryl-lower alkyl, cycloalkyl or hydroxy-lower alkyl; a pharmaceutically acceptable ester derivative thereof in which free hydroxy groups are esterified in form of a pharmaceutically acceptable ester; or a pharmaceutically acceptable salt thereof.

2. A compound according to claim 1 of formula I wherein R represents hydrogen or lower alkyl; $R^1$ represents cycloalkyl-lower alkyl; or $R^1$ represents aryl-lower alkyl wherein aryl represents pyridyl, thienyl, naphthyl, phenyl, phenyl substituted by one or two substituents selected from halogen, trifluoromethyl, lower alkoxy, hydroxy and lower alkyl, or phenyl substituted by a substituent -W-Z in which W represents a direct bond, lower alkylene, lower alkenylene, thio-lower alkylene or oxy-lower alkylene, and Z represents cyano, carboxy, carboxy derivatized in the form of a pharmaceutically acceptable ester or carboxy derivatized in the form of a pharmaceutically acceptable amide; or $R^1$ represents a substituent of the formula B

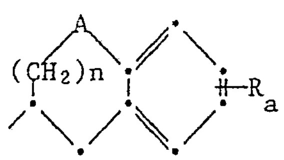

(B)

in which A represents methylene, oxygen or sulfur, n represents zero or one, and $R_a$ represents hydrogen, lower alkyl, lower alkoxy, halogen or -W-Z as defined above; $R^2$ represents hydrogen or lower alkyl; $R^3$ represents hydrogen or hydroxy; $R^4$ represents hydrogen, lower alkyl, cycloalkyl, hydroxy-lower alkyl, or aryl-lower alkyl in which aryl represents pyridyl, thienyl or phenyl; a pharmaceutically acceptable ester derivatives thereof in which one or more free hydroxy groups are esterified in form of a pharmaceutically acceptable ester; or a pharmaceutically acceptable salt thereof.

3. A compound according to claim 1 of formula Ia

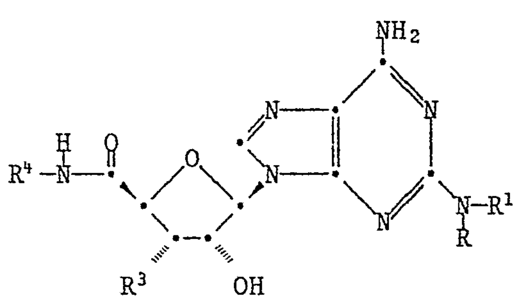

(Ia)

wherein R represents hydrogen or lower alkyl; $R^1$ represents cycloalkyl-lower alkyl; or $R^1$ represents aryl-lower alkyl in which aryl represents thienyl, pyridyl, phenyl or phenyl monosubstituted by halogen, trifluoromethyl, lower alkoxy, hydroxy, lower alkyl, or by a substituent -W-Z in which W represents a direct bond, lower alkylene, thio-lower alkylene or oxy-lower alkylene, and Z represents cyano, carboxy, lower alkoxycarbonyl, carbamoyl, N-mono- or N,N-di-lower alkylcarbamoyl; or $R^1$ represents a substituent of

the formula B'

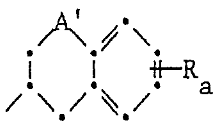

(B')

in which A' represents a direct bond, methylene, oxygen or sulfur, and $R_a$ represents hydrogen, lower alkyl, lower alkoxy, hydroxy, halogen or -W-Z as defined above; $R^3$ represents hydrogen or hydroxy; and $R^4$ represents hydrogen, lower alkyl, cycloalkyl or hydroxy-lower alkyl; a pharmaceutically acceptable prodrug ester derivative thereof in which one or more free hydroxy groups are esterified in form of a pharmaceutically acceptable ester; or a pharmaceutically acceptable salt thereof.

4. A compound according to any of preceding claims of formula I or Ia, wherein $R^3$ represents hydroxy, a pharmaceutically acceptable prodrug ester derivative thereof in which one or more free hydroxy groups are esterified in form of a pharmaceutically acceptable ester; or a pharmaceutically acceptable salt thereof.

5. A compound according to claim 3 of formula Ia wherein $R^3$ represents hydroxy; $R^4$ represents lower alkyl, cyclopropyl or hydroxy-lower alkyl; and R, $R^1$, A' and $R_a$ have meaning as defined in said claim; a pharmaceutically acceptable prodrug ester derivative thereof in which free hydroxy groups are esterified in form of a pharmaceutically acceptable ester; or a pharmaceutically acceptable salt thereof.

6. A compound according to claim 3 of formula II

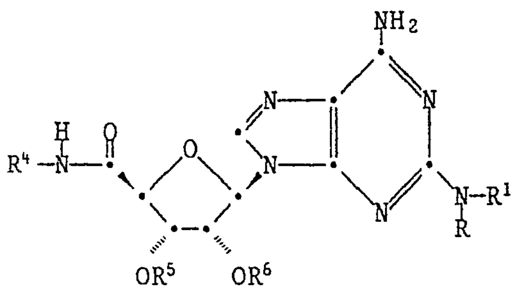

(II)

wherein R represents hydrogen or $C_1$-$C_4$-alkyl; $R^1$ represents ($C_5$- or $C_6$)-cycloalkyl-$C_1$-$C_4$-alkyl, or $R^1$ represents aryl-$C_1$-$C_4$-alkyl in which aryl represents 2- or 3-thienyl, 2-, 3-, or 4-pyridyl, phenyl, or phenyl monosubstituted by halogen, trifluoromethyl, lower alkoxy, lower alkyl or by a substituent -W-Z in which W represents a direct bond, $C_1$-$C_4$-alkylene, thio-$C_1$-$C_4$-alkylene or oxy-$C_1$-$C_3$-alkylene and Z represents cyano, carboxy, lower alkoxycarbonyl, carbamoyl, N-mono- or N,N-di-lower alkylcarbamoyl; $R^4$ represents $C_1$-$C_4$-alkyl, cyclopropyl or hydroxy-$C_2$-$C_4$-alkyl; $R^5$ and $R^6$ represent hydrogen, lower alkanoyl, lower alkoxy-lower alkanoyl, aroyl, carbamoyl, mono- or di-lower alkylcarbamoyl; or a pharmaceutically acceptable salt thereof.

7. A compound according to claim 6 of formula II wherein R represents $C_1$-$C_3$-alkyl or hydrogen; $R^1$ represents $-CH_2-CH_2-$(cyclohexyl or cyclopentyl); or $R^1$ represents $-CH_2-CH_2-$aryl in which aryl represents 2- or 3-pyridyl, phenyl, or phenyl monosubstituted by a substituent $-CH_2-CH_2-Z$ or $-OCH_2-Z$ in which Z represents cyano, carboxy, lower alkoxycarbonyl, carbamoyl, N-mono- or N,N-di-lower alkylcarbamoyl; $R^4$ represents ethyl or hydroxyethyl; $R^5$ and $R^6$ represent hydrogen, lower alkanoyl or lower alkozy-$C_2$-$C_4$-alkanoyl; or a pharmaceutically acceptable salt thereof.

8. A compound according to claim 6 of formula II wherein R represents hydrogen or methyl; $R^1$ represents cyclohexylethyl; or $R^1$ represents 2-phenylethyl, 2-(2-pyridyl)ethyl or 2-phenylethyl substituted in the para position by $-CH_2CH_2Z$ in which Z represents carboxy, lower alkoxycarbonyl, carbamoyl or mono-lower alkylcarbamoyl; $R^4$ represents ethyl; $R^5$ and $R^6$ represent hydrogen; or a pharmaceutically acceptable salt thereof.

9. A compound according to claim 6 of formula IIa

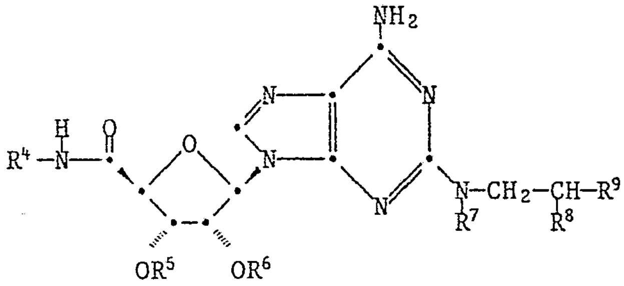

(IIa)

wherein $R^4$ represents ethyl; $R^5$ and $R^6$ represent hydrogen or lower alkanoyl; $R^7$ represents hydrogen or methyl; $R^8$ represents hydrogen or methyl; $R^9$ represents cyclohexyl, phenyl, or phenyl monosubstituted by halogen, trifluoromethyl, lower alkyl, lower alkoxy or $-CH_2CH_2-Z$ in which Z represents carboxy or lower alkoxycarbonyl; or a pharmaceutically acceptable salt thereof.

10. A compound according to claim 6 being 2-(2-cyclohexylethyl)-adenosine-5′-(N-ethyl)-carboxamide or a pharmaceutically acceptable salt thereof.

11. A compound according to claim 6 being 2-(2-phenethylamino)-adenosine-5′-(N-ethyl)-carboxamide or a pharmaceutically acceptable salt thereof.

12. A compound according to claim 6 being 2-[p-(2-carboxyethyl)-2-phenethylamino]-adenosine-5′-(N-ethyl)-carboxamide or a pharmaceutically acceptable salt thereof.

13. A compound according to claim 6 being 2[p-(carboxymethoxy)-2-phenethylamino]-adenosine-5′-(N-ethyl)-carboxamide or a pharmaceutically acceptable salt thereof.

14. A compound according to claim 6 being 2-(p-methoxy-2-phenethylamino)-adenosine-5′-(N-ethyl)-carboxamide or a pharmaceutically acceptable salt thereof.

15. A compound according to claim 6 being 2(p-chloro-2-phenethylamino)-adenosine-5′-(N-ethyl)-carboxamide or a pharmaceutically acceptable salt thereof.

16. A compound according to claim 6 being 2(p-fluoro-2-phenethylamino)-adenosine-5′-(N-ethyl)-carboxamide or a pharmaceutically acceptable salt thereof.

17. A pharmaceutical composition comprising a compound claimed in any one of claims 1 to 16, in admixture or conjunction with a pharmaceutically suitable carrier.

18. A compound claimed in any one of claims 1 to 16 for use in a therapeutic method of treating humans and animals.

19 A compound claimed in any one of claims 1 to 16 as adenosine-2 receptor agonist.

20. A compound claimed in any one of claims 1 to 16 as antipsychotic agent.

21. A compound claimed in any one of claims 1 to 16 as antihypertensive agent.

22. The use of a compound of any one of claims 1 to 16 in producing of a pharmaceutical composition.

23. The use of a compound of any one of claims 1 to 16 for the manufacture of a pharmaceutical composition for application as antipsychotic or antihypertensive agent.

24. Process for the manufacture of a pharmaceutical composition wherein a product as claimed in any of claims 1 to 16 is worked up with a pharmaceutical carrier.

25. Process for the manufacture of compounds of formula I shown in claim 1, in which formula all the symbols have the meanings given in claim 1, salts and stereoisomers of all these compounds, which consists in a) condensing a compound of the formula III

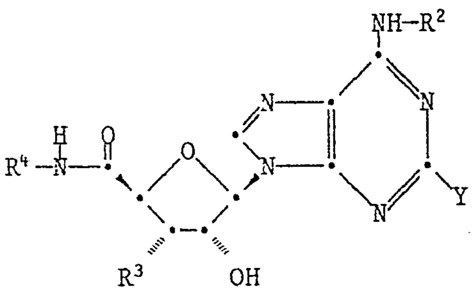

(III)

wherein $R^2$, $R^3$ and $R^4$ have meaning as defined in said claim and Y represents a leaving group, with a compound of the formula IV

$R^1$-NH-R (IV)

wherein R and $R^1$ have meaning as defined in said claim; and, if required, temporarily protecting any interfering reactive group(s) in the starting materials and then subsequently removing the protecting groups to yield a resulting compound of formula I; or in b) condensing a compound of the formula V

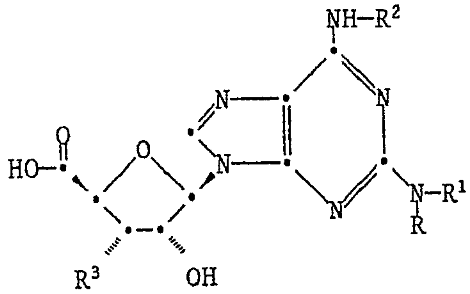

(V)

wherein $R^1$, $R^2$ and $R^3$ having meaning as defined in said claim, or a reactive functional derivative thereof, with an amine of the formula VI

$R^4$-$NH_2$ (VI)

wherein $R^4$ has meaning as defined in said claim; and, if required, temporarily protecting any interfering reacting group(s) in the starting materials and then subsequently removing the protecting groups to yield a resulting compound of formula I; and in any of these processes, if desired, converting a resulting compound of formula I into another compound of the invention, and if desired, converting a resulting free compound into a salt or a resulting salt into a free compound or into another salt, and if required, separating any mixture of isomers or racemates obtained into the single isomers or racemates, and if required, resolving a racemate into the optical antipodes.

26. The new compounds obtainable according to claim 25.